# EUROPÄISCHE PATENTSCHRIFT

(11) **EP 3 456 319 B1**
(45) Veröffentlichungstag und Bekanntmachung des Hinweises auf die Patenterteilung: **26.08.2026**
(21) Anmeldenummer: 17191288.4
(22) Anmeldetag: 15.09.2017
(51) Int. Cl.: A61K 45/06, A61K 9/20, A61K 31/522, A61K 31/155

(54) **DPP-4-INHIBITOR-ZUSAMMENSETZUNG**
DPP-4-INHIBITOR-COMPOSITION
COMPOSITION INHIBITEUR DE LA DPP-4

(43) Veröffentlichungstag der Anmeldung: 20.03.2019
(73) Patentinhaber: STADA ARZNEIMITTEL AG, 61118 Bad Vilbel (DE)
(72) Erfinder: Schönborn, Jessica, 61118 Bad Vilbel (DE)
(74) Vertreter: Kernebeck, Thomas

(56) Entgegenhaltungen:
- WO-A1-2014/080383
- WO-A2-2009/121945
- CN-A- 103 239 719
- US-A1- 2015 283 248
- BERTOL CHARISE DALLAZEM ET AL: "Physicochemical characterization of dipeptidyl peptidase-4 inhibitor alogliptin in physical mixtures with excipients", JOURNAL OF THERMAL ANALYSIS AND CALORIMETRY, KLUWER, DORDRECHT, NL, vol. 130, no. 3, 30 June 2017 (2017-06-30), pages 1575 - 1584, XP036365021, ISSN: 1388-6150, [retrieved on 20170630], DOI: 10.1007/S10973-017-6543-6
- S. SHANTIKUMAR ET AL: "Compatibility study between sitagliptin and pharmaceutical excipients used in solid dosage forms", JOURNAL OF THERMAL ANALYSIS AND CALORIMETRY, vol. 115, no. 3, 30 July 2013 (2013-07-30), NL, pages 2423 - 2428, XP055452986, ISSN: 1388-6150, DOI: 10.1007/s10973-013-3329-3
- ANONYMOUSLY: "Formulation comprising Metformin in combination with DPP-4 inhibitors", 27 March 2015 (2015-03-27), pages 1 - 6, XP055890205, Retrieved from the Internet <URL:http://null/IPCOM/000241112>

## Beschreibung

Die vorliegende Erfindung betrifft eine pharmazeutische Zusammensetzung, umfassend einen DPP-4-Inhibitor als ersten Wirkstoff sowie einen sauer reagierenden zweiten Wirkstoff.

Dipeptidylpeptidase (DPP)-4-Inhibitoren sind Substanzen, die insbesondere bei Behandlung des Diabetes mellitus Typ 2 eingesetzt werden können. Die metabolischen Wirkungen der Inhibitoren beruhen dabei auf der selektiven reversiblen kompetitiven Hemmung der Serinprotease Dipeptidylpeptidase-4 (DPP-4), die das körpereigene Inkretin GLP-1 (*Glucagon-like peptide-1*) abbaut. GLP-1, das nach einer Mahlzeit im Dünndarm in die Blutzirkulation ausgeschüttet wird, stimuliert beispielsweise die Glucose-abhängige Freisetzung von Insulin aus den Betazellen der Bauchspeicheldrüse, reduziert die Glucagonsekretion aus den Alphazellen der Bauchspeicheldrüse - und führt dadurch zu einer verminderten Glucoseproduktion in der Leber - und verlangsamt ferner die Magenentleerung in den Darm. Durch die Inhibition des Enzyms DPP-4 steigt die Konzentration des Inkretins GLP-1 an und die genannten antidiabetischen Wirkungen werden dadurch verstärkt.

DPP-4-Inhibitoren sind daher zur Behandlung von Diabetes mellitus Typ 2 zugelassen, wobei sie beispielsweise mit Biguaniden, Thiazolidindionen, Sulfonylharnstoffen und teilweise auch mit Insulin kombiniert werden können.

DPP-4-Inhibitoren an sich weisen eine verhältnismäßig hohe chemische Stabilität auf. In Kombinationsformulierungen zusammen mit insbesondere sauer reagierenden pharmazeutischen Wirkstoffen jedoch zeigen DPP-4-Inhibitoren eine nicht zu vernachlässigende Instabilität. Zur Lösung dieser Problematik schlägt WO 2009/121945 vor, einer Kombinationsformulierung von einem DPP-4-Inhibitor mit einem weiteren pharmazeutischen Wirkstoff ein nucleophiles und/oder basisches Agens zuzusetzen, insbesondere L-Arginin. Allerdings gestaltet es sich vergleichsweise schwierig, L-Arginin auf verfahrenstechnische einfache Weise wirkungsvoll in eine entsprechende Kombinationsformulierung einzuarbeiten.

In der WO 2014/080383 wird eine pharmazeutische Formulierung beschrieben, welche enthält:
i. einen DPP-4-Inhibitor;
ii. einen zusätzlichen antidiabetischen Wirkstoff;
iii. zumindest ein alkalisierendes Agens; und
iv. ein oder mehrere pharmazeutische Hilfsstoffe,
wobei die pharmazeutische Zusammensetzung ein alkalisierendes Agens enthält, das unterschiedlich ist zu basischen Aminosäuren.

In der anonymen Veröffentlichung auf ip.com, Veröffentlichungsnummer 000241112, "Formulation comprising Metformin in combination with DPP-4-Inhibitors", 27. März 2015 wird eine pharmazeutische Formulierung beschrieben, welche Metformin in Kombination mit einem DPP-4-Inhibitor enthält.

Aufgabe der vorliegenden Erfindung ist es daher, eine pharmazeutische Zusammensetzung, umfassend einen DPP-4-Inhibitor als ersten Wirkstoff sowie einen sauer reagierenden zweiten Wirkstoff, bereitzustellen, die durch eine relative gute Stabilität bezüglich des DPP-4-Inhibitors gekennzeichnet ist und sich verfahrenstechnisch einfach und damit kostengünstig herstellen lässt.

Diese Aufgabe wird gelöst durch eine pharmazeutische Zusammensetzung der eingangs genannten Art, umfassend einen DPP-4-Inhibitor als ersten Wirkstoff sowie einen sauer reagierenden zweiten Wirkstoff, wobei der erste Wirkstoff und der zweite Wirkstoff in der Zusammensetzung in Form einer Mischung zusammen mit einem Stearat vorliegen, wobei die Zusammensetzung ein Feuchtgranulat ist.

Überraschender Weise wurde festgestellt, dass ein DPP-4-Inhibitor in Mischung mit einem sauer reagierenden zweiten Wirkstoff und mit einem Stearat - sowie optional mit einem weiteren pharmazeutischen Hilfsstoff oder mit weiteren pharmazeutischen Hilfsstoffen - eine verhältnismäßig hohe Stabilität aufweist. Dabei kann das Stearat auf verhältnismäßig einfache Art und Weise, beispielsweise durch Untermischen des pulverförmigen Stearats, in die Zusammensetzung eingearbeitet werden, weshalb die erfindungsgemäße Zusammensetzung verhältnismäßig einfach und damit kostengünstig herstellbar ist.

Unter "Stabilität" wird in diesem Zusammenhang die von der Arbeitsgemeinschaft für pharmazeutische Verfahrenstechnik (APV) erarbeitete Definition verstanden, nach welcher "Stabilität" die spezifikationsgerechte Qualität des Arzneimittels bis zum Ende der vom Hersteller festgelegten Laufzeit bedeutet. Die Qualität des Arzneimittels wird dabei durch den Wirkstoffgehalt und die Reinheit, die sensorisch wahrnehmbaren, physikalisch-chemischen und die mikrobiologischen Eigenschaften bestimmt, wobei der Wirkstoffgehalt bis zum Ende der Laufzeit 90 % des deklarierten Wertes nicht unterschreiten soll.

Darüber hinaus weist die erfindungsgemäße Zusammensetzung eine vergleichsweise einfache Formulierung mit wenigen Komponenten auf, da Stearate in pharmazeutischen Zusammensetzungen ohnehin beispielsweise als Schmiermittel zugegen sind, was die Kosten für die Herstellung der erfindungsgemäßen Zusammensetzung weiter vermindert.

Ferner sind Stearate gängige, weitgehend inerte pharmazeutische Hilfsstoffe, bezüglich derer keine nachteiligen Inkompatibilitäten im Hinblick auf die vorliegend in Rede stehenden Wirkstoffe und im Hinblick auf andere pharmazeutische Hilfsstoffe beobachtet werden konnten.

In der erfindungsgemäßen Zusammensetzung ist es vorgesehen, dass der erste Wirkstoff, der zweite Wirkstoff und das Stearat in der Zusammensetzung in Form einer innigen Mischung vorliegen. Es konnte festgestellt werden, dass der DPP-4-Inhibitor in der erfindungsgemäßen Zusammensetzung umso stabiler ist, je besser die Durchmischung von DPP-4-Inhibitor, sauer reagierendem Wirkstoff und Stearat ist.

Gemäß einer weiter bevorzugten Ausführungsform der erfindungsgemäßen Zusammensetzung ist es vorgesehen, dass das Stearat in der Zusammensetzung gleichmäßig verteilt enthalten ist. Es wurde festgestellt, dass der DPP-4-Inhibitor umso stabiler ist, desto gleichmäßiger verteilt das Stearat in der erfindungsgemäßen Zusammensetzung enthalten ist.

Weiter ist bei der erfindungsgemäßen Zusammensetzung vorgesehen, dass das Stearat in der Zusammensetzung homogen verteilt enthalten ist.

Entsprechend einer weiter bevorzugten Ausführungsform der erfindungsgemäßen Zusammensetzung ist es vorgesehen, dass das Stearat ausgewählt ist aus der Gruppe bestehend aus Alkalimetall- und Erdalkalimetallstearaten und vorzugsweise aus der Gruppe bestehend aus Calcium- und Magnesiumstearat, wobei Magnesiumstearat ganz besonders bevorzugt ist. Es konnte festgestellt werden, dass die genannten Stearate, aber insbesondere Magnesiumstearat geeignet ist, den DPP-4-Tnhibitor vor den Wirkungen des sauer reagierenden Wirkstoffs hinsichtlich chemischer Degradation zu stabilisieren.

Gemäß einer weiter bevorzugten Ausführungsform der erfindungsgemäßen Zusammensetzung ist es vorgesehen, dass das Stearat in der Zusammensetzung in eine Menge von 2,5 mg bis 50 mg enthalten ist, vorzugsweise in einer Menge von 5 mg bis 25 mg und mehr bevorzugt in einer Menge von 5 mg bis 15 mg. Es konnte festgestellt werden, dass das Stearat in den genannten Mengenbereichen den DPP-4-Inhibitor in der erfindungsgemäßen Zusammensetzung vor den Wirkungen des sauer reagierenden Wirkstoffs hinsichtlich chemischen Abbaus ausreichend stabilisieren kann. Je nach Natur des DPP-4-Inhibitors, des sauer reagierenden Wirkstoffs, des Stearats und möglicher weiterer pharmazeutischer Hilfsstoffe kann es vorgesehen sein, die Menge an Stearat in der Zusammensetzung auf einen Maximalwert zu begrenzen, um die Freisetzung des ersten und des zweiten Wirkstoffs im beispielsweise menschlichen Körper nicht nachteilig zu beeinflussen.

DPP-4-Inhibitoren, die im Stand der Technik auch als Gliptine bezeichnet werden, weisen in der Regel eine intramolekulare freie primäre oder sekundäre Aminogruppe auf. Nach einer weiter bevorzugten Ausführungsform der erfindungsgemäßen Zusammensetzung ist es daher vorgesehen, dass der DPP-4-Inhibitor eine intramolekulare freie primäre oder sekundäre Aminogruppe aufweist.

Entsprechend einer weiter bevorzugten Ausführungsform der erfindungsgemäßen Zusammensetzung ist es vorgesehen, dass der DPP-4-Inhibitor ausgewählt ist aus der Gruppe bestehend aus Alogliptin, Denagliptin, Linagliptin, Saxagliptin, Sitagliptin und Vildagliptin. Es konnte festgestellt werden, dass die genannten DPP-4-Inhibitoren in der erfindungsgemäßen Zusammensetzung vor den Wirkungen des sauer reagierenden Wirkstoffs hinsichtlich chemischen Abbaus ausreichend stabilisiert werden können.

Die vorstehend genannten Gliptine weisen die folgenden Strukturformeln auf, wobei in den Strukturformeln die Symbole R und S (CIP-Nomenklatur) die absolute Konfiguration angeben:

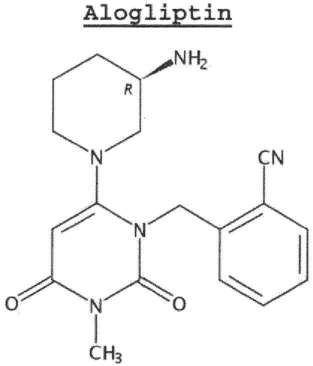

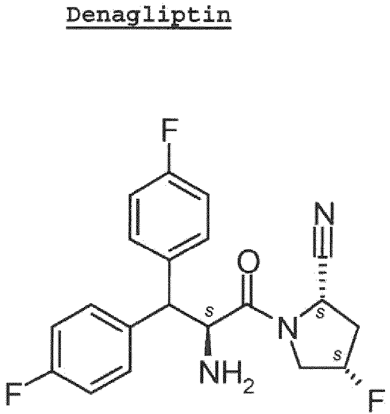

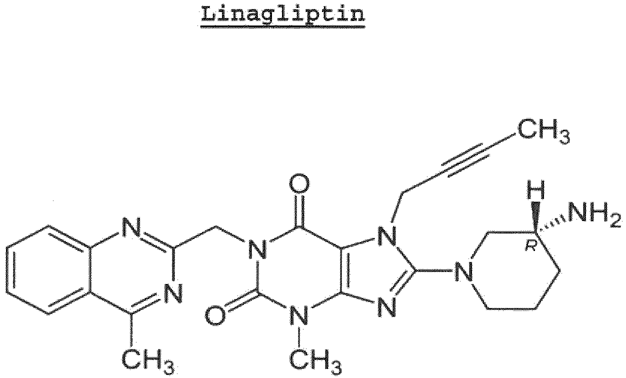

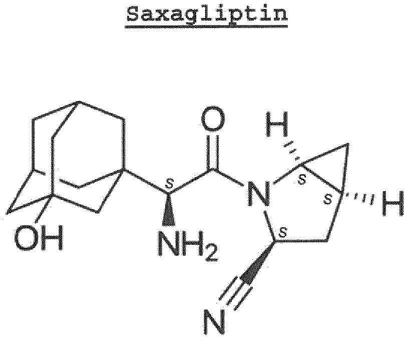

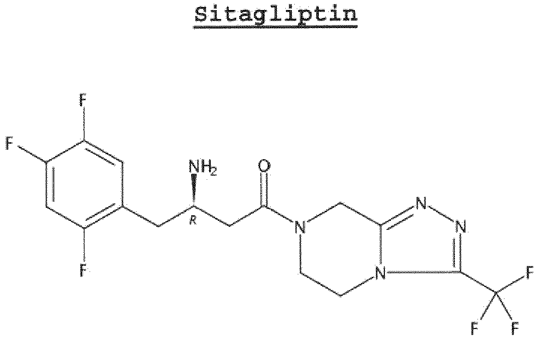

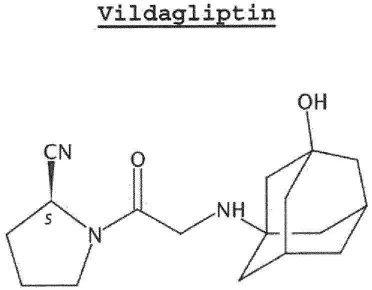

Gemäß einer weiter bevorzugten Ausführungsform der erfindungsgemäßen Zusammensetzung ist es vorgesehen, dass der DPP-4-Inhibitor Linagliptin ist.

Nach einer weiter bevorzugten Ausführungsform der erfindungsgemäßen Zusammensetzung ist es vorgesehen, dass der DPP-4-Inhibitor in der Zusammensetzung in einer Menge von 0,5 mg bis 100 mg enthalten ist, bevorzugt in einer Menge von 0,5 mg bis 50 mg und weiter bevorzugt in einer Menge von 0,5 mg, 1 mg, 2,5 mg, 5 mg, 10 mg oder 50 mg.

Entsprechend einer weiter bevorzugten Ausführungsform der erfindungsgemäßen Zusammensetzung ist es vorgesehen, dass der DPP-4-Inhibitor in der Zusammensetzung in einer Menge von 2,5 mg enthalten ist.

In der erfindungsgemäßen Zusammensetzung ist ein sauer reagierender zweiter pharmazeutischer Wirkstoff enthalten. Unter "sauer reagierend" wird dabei erfindungsgemäß verstanden, dass eine Lösung (bei leichtlöslichen Wirkstoffen), eine Dispersion (bei schwerlöslichen Wirkstoffen) bzw. eine Lösung/Dispersion (bei mäßig löslichen Wirkstoffen) von 0,5 g des Wirkstoffs in 10 ml destilliertem Wasser bei einer Temperatur von 25 °C einen pH-Wert von kleiner als 7,0 aufweist, bevorzugt einen pH-Wert von kleiner als 6,5 und mehr bevorzugt einen pH-Wert von kleiner als 6,0.

Erfindungsgemäß bevorzugt handelt es sich bei dem sauer reagierenden zweiten pharmazeutischen Wirkstoff um einen Wirkstoff, der zur Behandlung des Diabetes mellitus geeignet ist, vorzugsweise zur Behandlung des Diabetes mellitus Typ 2.

Gemäß einer weiter bevorzugten Ausführungsform der erfindungsgemäßen Zusammensetzung ist es vorgesehen, dass der zweite Wirkstoff ein Wirkstoff ist ausgewählt aus der Gruppe bestehend aus Biguaniden und Thiazolidindionen sowie deren pharmazeutisch annehmbare Salze.

Nach einer weiter bevorzugten Ausführungsform der erfindungsgemäßen Zusammensetzung ist es vorgesehen, dass der zweite Wirkstoff Metformin-Hydrochlorid ist oder Pioglitazon-Hydrochlorid, vorzugsweise Metformin-Hydrochlorid.

Entsprechend einer weiter bevorzugten Ausführungsform der erfindungsgemäßen Zusammensetzung ist es vorgesehen, dass der zweite Wirkstoff in der Zusammensetzung in einer Menge von 3 mg bis 1500 mg enthalten ist, vorzugsweise in einer Menge von 500 mg, 850 mg oder 1000 mg.

Gemäß einer weiter bevorzugten Ausführungsform der erfindungsgemäßen Zusammensetzung ist es vorgesehen, dass der erste Wirkstoff und das Stearat in der Zusammensetzung in einem Mengenverhältnis von 1:1 bis 1:10 vorliegen, vorzugsweise in einem Mengenverhältnis von 1:2 bis 1:5 oder in einem Mengenverhältnis 1:1,2 bis 1:2,5. In den genannten Mengenverhältnissen kann das Stearat den DPP-4-Inhibitor in der erfindungsgemäßen Zusammensetzung vor den Wirkungen des sauer reagierenden Wirkstoffs hinsichtlich chemischen Abbaus signifikant stabilisieren.

Nach einer weiter bevorzugten Ausführungsform der erfindungsgemäßen Zusammensetzung ist es vorgesehen, dass der zweite Wirkstoff und das Stearat in der Zusammensetzung in einem Mengenverhältnis von 50:1 bis 300:1 vorliegen, vorzugsweise in einem Mengenverhältnis von 100:1 bis 200:1. In den genannten Mengenverhältnissen kann das Stearat den DPP-4-Inhibitor in der erfindungsgemäßen Zusammensetzung vor den Wirkungen des sauer reagierenden Wirkstoffs hinsichtlich chemischen Abbaus deutlich stabilisieren.

Entsprechend einer weiter bevorzugten Ausführungsform der erfindungsgemäßen Zusammensetzung ist es vorgesehen, dass die Zusammensetzung eine feste Zusammensetzung ist.

Die erfindungsgemäße Zusammensetzung ist ein Feuchtgranulat das erfindungsgemäß bevorzugt mittels im Stand der Technik üblicher Techniken und gegebenenfalls mittels geeigneter pharmazeutischer Hilfsstoffe zu Tabletten oder Kapseln weiterverarbeitet werden kann.

So kann das Granulat beispielsweise mit geeigneten pharmazeutischen Hilfsstoffen vermischt und die resultierende Mischung zu einer Tablette verpresst werden. Das Granulat bildet dann die innere Phase der Tablette - oder genauer gesagt des Tablettenkerns - während die zum Granulat hinzugesetzten pharmazeutischen Hilfsstoffe die äußere Phase der Tablette/ des Tablettenkerns

bilden. Gemäß der Erfindung ist es vorgesehen, dass die Zusammensetzung ein Feuchtgranulat ist.

Entsprechend einer weiter bevorzugten Ausführungsform der erfindungsgemäßen Zusammensetzung ist es vorgesehen, dass die Zusammensetzung eine feste Kombination von zwei Wirkstoffen ist, d.h. neben dem ersten Wirkstoff und dem zweiten Wirkstoff keinen weiteren pharmazeutischen Wirkstoff umfasst.

Gemäß der Erfindung ist es vorgesehen, dass die Zusammensetzung in Form eines Feuchtgranulats vorliegt, und
- 0,5 mg bis 20 mg Linagliptin;
- 300 mg bis 1500 mg Metformin-Hydrochlorid und
- 2,5 mg bis 20 mg Magnesiumstearat
umfasst.

Nach einer weiter bevorzugten Ausführungsform der erfindungsgemäßen Zusammensetzung ist es vorgesehen, dass die Zusammensetzung ferner einen oder mehrere pharmazeutische Hilfsstoffe umfasst ausgewählt aus der Gruppe bestehend aus Bindemitteln, Füllmitteln, Sprengmitteln, Fließregulierungsmitteln und Schmiermitteln.

Eine typische, durch die vorliegende Erfindung bereitgestellte pharmazeutische Zusammensetzung ist ein Feuchtgranulat, umfassend 2,5 mg des DPP-4-Inhibitors Linagliptin als ersten Wirkstoff sowie 500 mg, 850 mg oder 1000 mg Metformin-Hydrochlorid als sauer reagierenden zweiten Wirkstoff, wobei der erste Wirkstoff und der zweite Wirkstoff in Form einer innigen Mischung zusammen mit 1 mg bis 10 mg Magnesiumstearat in der Zusammensetzung vorliegen. Dieses Granulat kann in einer Tablette eingearbeitet oder in einer Kapsel abgefüllt sein.

Die vorliegende Erfindung betrifft ferner ein Verfahren zur Herstellung der erfindungsgemäßen Zusammensetzung, umfassend die Schritte des
- Bereitstellens des ersten Wirkstoffs, des zweiten Wirkstoffs und des Stearates;
- Vermischens des ersten Wirkstoffs, des zweiten Wirkstoffs und des Stearates, optional unter Zusatz eines oder mehrerer pharmazeutischer Hilfsstoffe.

Entsprechend einer bevorzugten ersten Alternative des erfindungsgemäßen Verfahrens ist es vorgesehen, dass das Vermischen erfolgt, indem
- der erste Wirkstoff in Pulverform, der zweite Wirkstoff in Pulverform und das Stearat in Pulverform miteinander vermischt werden.

Gemäß einer bevorzugten zweiten Alternative des erfindungsgemäßen Verfahrens ist es vorgesehen, dass das Vermischen erfolgt, indem
- der zweite Wirkstoff in Pulverform und das Stearat in Pulverform miteinander vermischt werden unter Erhalt einer Vormischung;
- die Vormischung mittels einer Granulierflüssigkeit, in welcher der erste Wirkstoff gelöst und/oder dispergiert enthalten ist, unter Erhalt eines Granulates granuliert wird.

Nach einer bevorzugten dritten Alternative des erfindungsgemäßen Verfahrens ist es vorgesehen, dass das Vermischen erfolgt, indem
- der zweite Wirkstoff zusammen mit dem Stearat in einer Flüssigkeit gelöst und/oder dispergiert wird und die resultierende Mischung getrocknet wird unter Erhalt einer Vormischung;
- die Vormischung mit einer Granulierflüssigkeit, in welcher der erste Wirkstoff gelöst und/oder dispergiert enthalten ist, unter Erhalt eines Granulates granuliert wird.

Gemäß einer weiter bevorzugten Ausführungsform des erfindungsgemäßen Verfahrens gemäß erster, zweiter oder dritter Alternative kann es vorgesehen sein, dass das Verfahren ferner den Schritt umfasst des
- Verarbeitens der Pulvermischung bzw. des Granulates zu einer Tablette oder Kapsel, optional unter Verwendung eines oder mehrerer pharmazeutischer Hilfsstoffe.

Die nachfolgenden Ausführungsbeispiele dienen der Erläuterung der Erfindung.

### Ausführungsbeispiel 1:

Es wurden Filmtabletten mit der in Tabelle 1 angegebenen Formulierung hergestellt:

**Tabelle 1:**

| | **1. Kerne** | | |
|---|---|---|---|
| **Granulat (Innenphase)** | | **Menge pro Tablette [mg]** | **Funktion der Substanz** |
| Linagliptin | | 2,50 | erster Wirkstoff |
| Metformin-Hydrochlorid | | 1000,00 | zweiter Wirkstoff |
| Magnesiumstearat | | 5,00 | Stearat zur Stabilisierung des ersten Wirkstoffs |
| Cellulose, gepulvert (Arbocel^{®} A300) | | 68,90 mg | Füllstoff |
| Copovidon (Kollidon^{®} VA 64 fine) | | 95,00 | Bindemittel |
| Wasser, gereinigt (im Endprodukt nicht mehr enthalten) | | 50,82 | Flüssigkeit für Granulierflüssigkeit |
| **Außenphase** | | | |
| Crospovidon (Kollidon^{®} CL) | | 23,60 | Sprengmittel |
| hochdisperses Siliciumdioxid (Aerosil^{®}) | | 5,00 | Fließregulierungsmittel |
| Magnesiumstearat | | 10,00 | Schmiermittel teils auch zur Stabilisierung des ersten Wirkstoffs |
| **Gewicht Tablettenkern** | | 1210,00 | |

| | **2. Film** | | |
|---|---|---|---|
| Opadry^{®} bestehend aus: | | 24,00 | Befilmungssystem |
| Hypromellose (E464), Talc (E553b), Titandioxid (E171), Macrogol (E1521), Eisenoxid rot (E 172) | | | |
| **Gesamtgewicht der Filmtablette** | | 1234,00 | |

Die Filmtabletten wurden wie folgt hergestellt: Metformin-Hydrochlorid, Magnesiumstearat, gepulverte Cellulose und Copovidon wurden mittels eines konventionellen Mischers ausgiebig miteinander vermischt unter Erhalt einer pulverförmigen Vormischung. Diese pulverförmige Vormischung wurde mittels einer Dispersion von Linagliptin in Wasser unter Verwendung einer herkömmlichen Granuliervorrichtung granuliert und anschließend getrocknet unter Erhalt eines Granulates als erfindungsgemäße Zusammensetzung.

Das so erhalte Granulat wurde mit Crospovidon, hochdispersem Siliciumdioxid und Magnesiumstearat vermischt und die so erhaltene Mischung mittels einer konventionellen Tablettenpresse zu Tablettenkernen verpresst.

Die so erhaltenen Tablettenkerne wurden in einer konventionellen Filmbeschichtungsvorrichtung durch Besprühen mit einer Beschichtungssuspension befilmt. Die Beschichtungssuspension wurde hergestellt durch Suspendieren eines kommerziell erhältlichen Befilmungssystems Opadry^{®} in Wasser.

Stabilitätsdaten der Tabletten:

| Lagerungsbedingungen: | Ausgangswert [%] | 1 Monat [%] | 3 Monate [%] | 6 Monate [%] |
|---|---|---|---|---|
| 40°C/75% r.h.; | | | | |
| Packmittel: | | | | |
| PVC/PVDC/Alu 250/180 | | | | |
| Blister | | | | |
| Verunreinigungen (gesamt) | 0.14 | 0.17 | 0.19 | 0.32 |

### Ausführungsbeispiel 2:

Es wurden Filmtabletten mit der in Tabelle 2 angegebenen Formulierung hergestellt:

**Tabelle 2:**

| | **1. Kerne** | | |
|---|---|---|---|
| **Granulat (Innenphase)** | | **Menge pro Tablette [mg]** | **Funktion der Substanz** |
| Linagliptin | | 2,50 | erster Wirkstoff |
| Metformin-Hydrochlorid | | 850,00 | zweiter Wirkstoff |
| Magnesiumstearat | | 4,25 | Stearat zur Stabilisierung des ersten Wirkstoffs |
| Cellulose, gepulvert (Arbocel^{®} A300) | | 58,19 mg | Füllstoff |
| Copovidon (Kollidon^{®} VA 64 fine) | | 80,75 | Bindemittel |
| | | | |
| Wasser, gereinigt (im Endprodukt nicht mehr enthalten) | | 43,20 | Flüssigkeit für Granulierflüssigkeit |
| **Außenphase** | | | |
| Crospovidon (Kollidon^{®} CL) | | 20,06 | Sprengmittel |
| hochdisperses Siliciumdioxid (Aerosil^{®}) | | 4,25 | Fließregulierungsmittel |
| Magnesiumstearat | | 8,50 | Schmiermittel teils auch zur Stabilisierung des ersten Wirkstoffs |
| **Gewicht Tablettenkern** | | 1028,50 | |

| | **2. Film** | | |
|---|---|---|---|
| Opadry^{®} bestehend aus: | | 20,50 | Befilmungssystem |
| Hypromellose (E464), Talc (E553b), Titandioxid (E171), Macrogol (E1521), Eisenoxid rot (E 172) Eisenoxid gelb (E 172) | | | |
| **Gesamtgewicht der Filmtablette** | | 1049,00 | |

Die Herstellung der Filmtabletten erfolgte analog Ausführungsbeispiel 1.

Stabilitätsdaten der Tabletten:

| Lagerungsbedingungen: | Ausgangswert [%] | 1 Monat [%] | 3 Monate [%] | 6 Monate [%] |
|---|---|---|---|---|
| 40°C/75% r.h.; | | | | |
| Packmittel: | | | | |
| PVC/PVDC/Alu 250/180 | | | | |
| Blister | | | | |
| | | | | |
| Verunreinigungen (gesamt) | 0.16 | 0.17 | 0.20 | 0.31 |

### Ausführungsbeispiel 3:

Es wurden Filmtabletten mit der in Tabelle 3 angegebenen Formulierung hergestellt:

**Tabelle 3:**

| | **1. Kerne** | | |
|---|---|---|---|
| **Granulat (Innenphase)** | | **Menge pro Tablette [mg]** | **Funktion der Substanz** |
| Linagliptin | | 2,50 | erster Wirkstoff |
| Metformin-Hydrochlorid + 0,5 % MagnesiumStearat (Firma: Wanbury) | | 1005,03 | zweiter Wirkstoff + Stearat zur Stabilisierung des ersten Wirkstoffs |
| Cellulose, gepulvert (Arbocel^{®} A300) | | 38,87 mg | Füllstoff |
| Copovidon (Kollidon^{®} VA 64 fine) | | 95,00 | Bindemittel |
| Wasser, gereinigt (im Endprodukt nicht mehr enthalten) | | 47,20 | Flüssigkeit für Granulierflüssigkeit |
| **Außenphase** | | | |
| Crospovidon (Kollidon^{®} CL) | | 23,60 | Sprengmittel |
| hochdisperses Siliciumdioxid (Aerosil^{®}) | | 5,00 | Fließregulierungsmittel |
| Magnesiumstearat | | 10,00 | Schmiermittel teils auch zur Stabilisierung des ersten Wirkstoffs |
| **Gewicht Tablettenkern** | | 1180,00 | |

| | **2. Film** | | |
|---|---|---|---|
| Opadry^{®} bestehend aus: | | 35,00 | Befilmungssystem |
| Hypromellose (E464), Talc (E553b), Titandioxid (E171), Macrogol (E1521), Eisenoxid rot (E 172) Eisenoxid gelb (E 172) | | | |
| **Gesamtgewicht der Filmtablette** | | 1215,00 | |

Die Herstellung der Filmtabletten erfolgte analog Ausführungsbeispiel 1.

Stabilitätsdaten der Tabletten:

| Lagerungsbedingungen: | Ausgangswert [%] | 1 Monat [%] | 3 Monate [%] | 6 Monate [%] |
|---|---|---|---|---|
| 40°C/75% r.h.; | | | | |
| Packmittel: | | | | |
| PVC/PVDC/Alu 250/180 Blister | | | | |
| Verunreinigungen (gesamt) | 0.15 | 0.15 | 0.16 | 0.34 |

### Ausführungsbeispiel 4:

Es wurden Filmtabletten mit der in Tabelle 4 angegebenen Formulierung hergestellt:

**Tabelle 4:**

| | **1. Kerne** | | |
|---|---|---|---|
| **Granulat (Innenphase)** | | **Menge pro Tablette [mg]** | **Funktion der Substanz** |
| Linagliptin | | 2,50 | erster Wirkstoff |
| | | | |
| Metformin-Hydrochlorid + 0,5 % Magnesium-Stearat (Firma: Wanbury) | | 854,27 | zweiter Wirkstoff + Stearat zur Stabilisierung des ersten Wirkstoffs |
| Cellulose, gepulvert (Arbocel^{®} A300) | | 32,67 mg | Füllstoff |
| Copovidon (Kollidon^{®} VA 64 fine) | | 80,75 | Bindemittel |
| Wasser, gereinigt (im Endprodukt nicht mehr enthalten) | | 40,12 | Flüssigkeit für Granulierflüssigkeit |

| **Außenphase** | | | |
|---|---|---|---|
| Crospovidon (Kollidon^{®} CL) | | 20,06 | Sprengmittel |
| hochdisperses Siliciumdioxid (Aerosil^{®}) | | 4,25 | Fließregulierungsmittel |
| Magnesiumstearat | | 8,5 | Schmiermittel teils zur Stabilisierung des ersten Wirkstoffs |
| **Gewicht Tablettenkern** | | 1003,00 | |

| | **2. Film** | | |
|---|---|---|---|
| Opadry^{®} bestehend aus: | | 30,00 | Befilmungssystem |
| Hypromellose (E464), Talc (E553b), Titandioxid (E171), Macrogol (E1521), Eisenoxid rot (E 172) Eisenoxid gelb (E 172) | | | |
| **Gesamtgewicht der Filmtablette** | | 1033,00 | |

Die Herstellung der Filmtabletten erfolgte analog Ausführungsbeispiel 1.

Stabilitätsdaten der Tabletten:

| Lagerungsbedingungen: | Ausgangswert [%] | 1 Monat [%] | 3 Monate [%] | 6 Monate [%] |
|---|---|---|---|---|
| 40°C/75% r.h.; | | | | |
| Packmittel: | | | | |
| PVC/PVDC/Alu 250/180 | | | | |
| Blister | | | | |
| Verunreinigungen (gesamt) | 0.13 | 0.16 | 0.17 | 0.32 |

## Patentansprüche

1. Pharmazeutische Zusammensetzung, umfassend einen DPP-4-Inhibitor als ersten Wirkstoff sowie einen sauer reagierenden zweiten Wirkstoff, wobei der erste Wirkstoff und der zweite Wirkstoff in der Zusammensetzung in Form einer Mischung zusammen mit einem Stearat vorliegen, wobei die Zusammensetzung ein Feuchtgranulat ist.

2. Zusammensetzung nach Anspruch 1, **dadurch gekennzeichnet, dass** der erste Wirkstoff, der zweite Wirkstoff und das Stearat in der Zusammensetzung in Form einer innigen Mischung vorliegen.

3. Zusammensetzung nach einem der voranstehenden Ansprüche, **dadurch gekennzeichnet, dass** das Stearat Magnesiumstearat ist.

4. Zusammensetzung nach einem der voranstehenden Ansprüche, **dadurch gekennzeichnet, dass** das Stearat in der Zusammensetzung in eine Menge von 2,5 mg bis 50 mg enthalten ist, vorzugsweise in einer Menge von 5 mg bis 25 mg.

5. Zusammensetzung nach einem der voranstehenden Ansprüche, **dadurch gekennzeichnet, dass** der DPP-4-Inhibitor Linagliptin ist.

6. Zusammensetzung nach einem der voranstehenden Ansprüche, **dadurch gekennzeichnet, dass** der DPP-4-Inhibitor in der Zusammensetzung in einer Menge von 0,5 mg bis 100 mg enthalten ist, bevorzugt in einer Menge von 0,5 bis 50 mg und weiter bevorzugt in einer Menge von 0,5 mg, 1 mg, 2,5 mg, 5 mg, 10 mg oder 50 mg.

7. Zusammensetzung nach einem der voranstehenden Ansprüche, **dadurch gekennzeichnet, dass** der zweite Wirkstoff Metformin-Hydrochlorid ist.

8. Zusammensetzung nach einem der voranstehenden Ansprüche, **dadurch gekennzeichnet, dass** der zweite Wirkstoff in der Zusammensetzung in einer Menge von 3 mg bis 1500 mg enthalten ist, vorzugsweise in einer Menge von 500 mg, 850 mg oder 1000 mg.

9. Zusammensetzung nach einem der voranstehenden Ansprüche, **dadurch gekennzeichnet, dass** der erste Wirkstoff und das Stearat in der Zusammensetzung in einem Mengenverhältnis von 1:1 bis 1:10 vorliegen, vorzugsweise in einem Mengenverhältnis von 1:2 bis 1:5 oder in einem Mengenverhältnis 1:1,2 bis 1:2,5.

10. Zusammensetzung nach einem der voranstehenden Ansprüche, **dadurch gekennzeichnet, dass** die Zusammensetzung in Form eines Feuchtgranulates vorliegt und
- 0,5 mg bis 20 mg Linagliptin;
- 300 mg bis 1500 mg Metformin-Hydrochlorid und
- 2,5 mg bis 20 mg Magnesiumstearat
umfasst.

11. Verfahren zur Herstellung einer Zusammensetzung nach einem der voranstehenden Ansprüche, umfassend die Schritte des
- Bereitstellens des ersten Wirkstoffs, des zweiten Wirkstoffs und des Stearates;
- Vermischens des ersten Wirkstoffs, des zweiten Wirkstoffs und des Stearates, optional unter Zusatz eines oder mehrerer pharmazeutischer Hilfsstoffe.

12. Verfahren nach Anspruch 11, **dadurch gekennzeichnet, dass** das Vermischen erfolgt, indem der zweite Wirkstoff in Pulverform und das Stearat in Pulverform miteinander vermischt werden unter Erhalt einer Vormischung;
- die Vormischung mittels einer Granulierflüssigkeit, in welcher der erste Wirkstoff gelöst und/oder dispergiert enthalten ist, unter Erhalt eines Granulates granuliert wird.

13. Verfahren nach Anspruch 12, **dadurch gekennzeichnet, dass** das Verfahren ferner den Schritt umfasst des
- Verarbeitens des Granulates zu einer Tablette oder zu einer Kapsel, optional unter Verwendung eines oder mehrerer pharmazeutischer Hilfsstoffe.

## Claims

1. Pharmaceutical composition comprising a DPP-4 inhibitor as first active ingredient and also an acidic second active ingredient, wherein the first active ingredient and the second active ingredient are present in the composition in the form of a mixture together with a stearate, the composition being a wet granulate.

2. Composition according to Claim 1, **characterized in that** the first active ingredient, the second active ingredient and the stearate are present in the composition in the form of an intimate mixture.

3. Composition according to any of the preceding claims, **characterized in that** the stearate is magnesium stearate.

4. Composition according to any of the preceding claims, **characterized in that** the stearate is present in the composition in an amount of from 2.5 mg to 50 mg, preferably in an amount of from 5 mg to 25 mg.

5. Composition according to any of the preceding claims, **characterized in that** the DPP-4 inhibitor is linagliptin.

6. Composition according to any of the preceding claims, **characterized in that** that the DPP-4 inhibitor is present in the composition in an amount of from 0.5 mg to 100 mg, preferably in an amount of from 0.5 to 50 mg and further preferably in an amount of 0.5 mg, 1 mg, 2.5 mg, 5 mg, 10 mg or 50 mg.

7. Composition according to any of the preceding claims, **characterized in that** the second active ingredient is metformin hydrochloride.

8. Composition according to any of the preceding claims, **characterized in that** the second active ingredient is present in the composition in an amount of from 3 mg to 1500 mg, preferably in an amount of 500 mg, 850 mg or 1000 mg.

9. Composition according to any of the preceding claims, **characterized in that** the first active ingredient and the stearate are present in the composition in an amount ratio of from 1:1 to 1:10, preferably in an amount ratio of from 1:2 to 1:5 or in an amount ratio of from 1:1.2 to 1:2.5.

10. Composition according to any of the preceding claims, **characterized in that** the composition is present in the form of a wet granulate and comprises:
- 0.5 mg to 20 mg of linagliptin;
- 300 mg to 1500 mg of metformin hydrochloride and
- 2.5 mg to 20 mg of magnesium stearate.

11. Process for producing a composition according to any of the preceding claims, comprising the steps of:
- providing the first active ingredient, the second active ingredient and the stearate;
- mixing the first active ingredient, the second active ingredient and the stearate, optionally with addition of one or more pharmaceutical excipients.

12. Process according to Claim 11, **characterized in that** the mixing takes place by mixing the second active ingredient in powder form and the stearate in powder form with one another to afford a premix;
- the premix is granulated by means of a granulation liquid in which the first active ingredient is dissolved and/or dispersed, to afford a granulate.

13. Process according to Claim 12, **characterized in that** the process also comprises the step of:
- processing the granulate into a tablet or capsule, optionally with the use of one or more pharmaceutical excipients.

## Revendications

1. Composition pharmaceutique comprenant un inhibiteur de DPP-4 comme première substance active ainsi qu'une deuxième substance active à réaction acide, la première substance active et la deuxième substance active étant présentes dans la composition sous la forme d'un mélange avec un stéarate, la composition étant un granulé humide.

2. Composition selon la revendication 1, **caractérisée en ce que** la première substance active, la deuxième substance active et le stéarate sont présents dans la composition sous la forme d'un mélange intime.

3. Composition selon l'une quelconque des revendications précédentes, **caractérisée en ce que** le stéarate est du stéarate de magnésium.

4. Composition selon l'une quelconque des revendications précédentes, **caractérisée en ce que** le stéarate est contenu dans la composition en une quantité allant de 2,5 mg à 50 mg, de préférence en une quantité allant de 5 mg à 25 mg.

5. Composition selon l'une quelconque des revendications précédentes, **caractérisée en ce que** l'inhibiteur de DPP-4 est la linagliptine.

6. Composition selon l'une quelconque des revendications précédentes, **caractérisée en ce que** l'inhibiteur de DPP-4 est contenu dans la composition en une quantité allant de 0,5 mg à 100 mg, de manière préférée en une quantité allant de 0,5 à 50 mg et par ailleurs de manière préférée en une quantité de 0,5 mg, 1 mg, 2,5 mg, 5 mg, 10 mg ou 50 mg.

7. Composition selon l'une quelconque des revendications précédentes, **caractérisée en ce que** la deuxième substance active est le chlorhydrate de metformine.

8. Composition selon l'une quelconque des revendications précédentes, **caractérisée en ce que** la deuxième substance active est contenue dans la composition en une quantité allant de 3 mg à 1500 mg, de préférence en une quantité de 500 mg, 850 mg ou 1000 mg.

9. Composition selon l'une quelconque des revendications précédentes, **caractérisée en ce que** la première substance active et le stéarate sont présents dans la composition en un rapport de quantité de 1:1 à 1:10, de préférence en un rapport de quantité de 1:2 à 1:5 ou en un rapport de quantité de 1:1,2 à 1:2,5.

10. Composition selon l'une quelconque des revendications précédentes, **caractérisée en ce que** la composition est présente sous la forme d'un granulé humide et comprend
- de 0,5 mg à 20 mg de linagliptine ;
- de 300 mg à 1500 mg de chlorhydrate de metformine et
- de 2,5 mg à 20 mg de stéarate de magnésium.

11. Procédé de fabrication d'une composition selon l'une quelconque des revendications précédentes, comprenant les étapes de
- la fourniture de la première substance active, de la deuxième substance active et du stéarate ;
- du mélange de la première substance active, de la deuxième substance active et du stéarate, en option en ajoutant un ou plusieurs excipients pharmaceutiques.

12. Procédé selon la revendication 11, **caractérisé en ce que** le mélange est effectué en mélangeant la deuxième substance active sous forme de poudre et le stéarate sous forme de poudre l'un avec l'autre en obtenant un prémélange ;
- le prémélange est granulé au moyen d'un liquide de granulation dans lequel la première substance active est dissoute et/ou dispersée, en obtenant un granulé.

13. Procédé selon la revendication 12, **caractérisé en ce que** le procédé comprend en outre l'étape de
- transformation du granulé en un comprimé ou en une gélule, en option en utilisant un ou plusieurs excipients pharmaceutiques.
